# EUROPEAN PATENT APPLICATION

(11) **EP 4 681 553 A1**
(43) Date of publication of application: **21.01.2026**
(21) Application number: 25189243.6
(22) Date of filing: 14.07.2025
(51) Int. Cl.: A23L 33/135, A61K 31/716, A61K 31/728, A61K 35/747, A61K 36/064, A61K 36/28

(54) **COMPOSITION FOR USE IN SUPPORTING AND/OR IMPROVING INTESTINAL HEALTH AND INTESTINAL FLORA**

(30) Priority: 15.07.2024 BE 202405459
(71) Applicant: Ventribio BV, 3960 Bree (BE)
(72) Inventor: SULS, Marc, 3960 Bree (BE); BROECKX, Sarah, 3960 Bree (BE); SPAAS, Jan, 3960 Bree (BE); BOOGAERTS, Hélène, 3960 Bree (BE)
(74) Representative: Brantsandpatents bv

(57) **Abstract**

The present invention relates to a composition for use in supporting and/or improving gut health and gut flora, wherein a recommended daily dose of the composition comprises at least a probiotic component comprising *Saccharomyces boulardii,* and at least three *Lactobacillus* strains, and a prebiotic component comprising at least two different sources of soluble fibers, wherein the composition further comprises hyaluronic acid and/or a salt thereof.

## Description

### TECHNICAL FIELD

The invention relates to a composition for use in the support and/or improvement of gut health and gut flora.

### PRIOR ART

A balanced gut flora is crucial for good digestion and general health, but this balance can be disrupted by factors such as improper diet, stress, and the use of antibiotics. As a result, harmful bacteria can gain the upper hand, leading to a disturbed digestion and the aforementioned symptoms.

Another significant problem is the integrity of the intestinal barrier. A weakened intestinal barrier can lead to a "leaky gut," wherein unwanted substances can enter the bloodstream and cause an immune response. This can result in chronic inflammation and associated health problems such as food sensitivities, autoimmune diseases, and metabolic disorders. In addition, there is a growing need for effective solutions to improve the hydration of the intestinal wall. A well-hydrated intestinal wall is essential for maintaining the barrier function, promoting the absorption of nutrients, and supporting a healthy gut flora. Insufficient hydration can lead to a dry and irritated intestinal wall, which further undermines overall intestinal health.

Despite the availability of various prebiotic and probiotic supplements, there is still a need for advanced formulations that can effectively address these problems. Specifically, there is a need for compositions that not only stimulate the growth of beneficial gut bacteria, but also contribute to strengthening the intestinal barrier and improving the hydration of the intestinal wall. This combined approach can have a significant impact on the overall health and well-being of individuals by supporting intestinal health on multiple fronts.

The invention aims to provide a solution for at least one of the aforementioned problems.

### SUMMARY OF THE INVENTION

The invention relates to a composition for use according to claim 1. The composition is optimized in such a way that it has a positive influence on gut health and gut flora.

In particular, the invention relates to a composition for use in the support and/or improvement of gut health and gut flora, wherein a recommended daily dose of the composition comprises at least the following ingredients:
- a probiotic component comprising:
   o Saccharomyces boulardii, in an amount of at least 10⁹ colony-forming units (CFU),
   ∘ at least three Lactobacillus strains, wherein the Lactobacillus strains are chosen from the group consisting of Lactobacillus casei, Lactobacillus plantarum, Lactobacillus rhamnosus, Lactobacillus acidophilus, and Lactobacillus paracasei, wherein the total amount of Lactobacillus in the daily dose is between 10⁸ and 10¹⁰ colony-forming units (CFU),
- a prebiotic component comprising at least two different sources of soluble fibers, wherein at least one source is of microbial origin,
wherein the composition further comprises hyaluronic acid and/or a salt thereof, wherein a recommended daily dose of the composition comprises the hyaluronic acid and/or the salt thereof in an amount of at least 5 mg.

The composition contains a carefully selected mix of ingredients that act on intestinal health on multiple fronts. Our solution provides a synergistic combination of probiotics, prebiotics, and hyaluronic acid, which jointly contribute to a balanced gut microbiome, the strengthening of the intestinal barrier, and the optimization of the hydration of the intestinal wall. This leads to a reduction of digestive complaints, a strengthening of the immune function, and a promotion of overall well-being.

It has been found that the addition of hyaluronic acid and soluble fibers not only provides for the strengthening of the intestinal barrier and the optimization of the hydration of the intestinal wall, but also promotes the colonization of the probiotic component and thus also the efficacy of the composition.

The composition as described herein exhibits improved microbial growth compared to the hyaluronic acid and fiber-free conditions. The addition of hyaluronic acid with soluble fibers such as beta-glucans, Inavea Baobab, acacia, and artichoke in the composition improves the growth of Lactobacillus and Saccharomyces.

Furthermore, the hyaluronic acid and soluble fibers cause a shift from pathogenic microbes to probiotic microbes in the intestine, which in turn contributes to the support and improvement of gut health and gut flora.

### DETAILED DESCRIPTION

Unless otherwise defined, all terms used in the description of the invention, including technical and scientific terms, have the meaning as commonly understood by a person skilled in the art to which the invention pertains. For a better understanding of the description of the invention, the following terms are explained explicitly.

As used herein, "administration" and "dosing" refer to the manner in which an active agent, or a composition containing it, is provided to a subject.

Administration can be effected via various routes known in the art, such as oral and non-oral methods.

As used herein, "subject" refers to a mammal that may benefit from the administration of a composition or method as described herein. Usually the subject will be a human. Additionally, the subject may also be an animal, preferably a mammal.

As used herein, "oral administration" or "oral dosing" refers to a route of administration that can be achieved by swallowing, chewing, or sucking an oral dosage form comprising the drug. Examples of known oral dosage forms are tablets, capsules, pills, powders, granules, liquids, syrups, elixirs, confectionery, or other foodstuffs, etc.

As used herein, "probiotic component" refers to a part of the composition that contains living microorganisms to which a health-promoting effect is attributed, and which in the present context are beneficial for intestinal health.

As used herein, "colony-forming units (CFU)" refers to the number of living bacteria or yeasts in the composition. "Colony-forming units (CFU)" is synonymous with "viable count." The viable count can be determined by applying a small amount of the product to be examined onto a petri dish with a selective medium and incubating it at specific temperatures, depending on the species expected. After a few days, the colonies formed are counted. Every viable microorganism will then have formed a colony.

As used herein, "prebiotic component" refers to a part of the composition that contains non-digestible food ingredients which selectively stimulate the growth and/or activity of one or more species of bacteria in the large intestine, and thereby promote the health of the host, such as soluble fibers.

As used herein, "soluble fibers" refers to non-digestible parts of plant food (fibers) that are broken down by bacteria in the large intestine.

As used herein, "microbial origin" refers to fibers that are derived from microorganisms.

As used herein, "formulation" and "composition" can be used interchangeably and refer to a combination of two or more elements or substances. In some embodiments, a composition can contain an active agent in combination with a carrier or other excipients, adjuvants, etc.

In this document, "a" and "the" refer to both the singular and the plural, unless the context presupposes otherwise. For example, "a segment" means one or more segments.

The terms "comprise," "comprising," "consist of," "consisting of," "provided with," "have," "having," "include," "including," "contain," "containing" are synonyms and are inclusive or open terms that indicate the presence of what follows, and which do not exclude or prevent the presence of other components, characteristics, elements, members, steps, as known from or disclosed in the prior art.

Quoting numeric intervals by the endpoints includes all integers, fractions, and/or real numbers between the endpoints, including those endpoints.

In a first aspect, the invention relates to a composition for use in the support and/or improvement of gut health and gut flora. Supporting and improving intestinal health is understood to mean the process whereby a healthy balance of the gut microbiota is promoted and the overall health of the intestinal tract is improved. Improved intestinal health can manifest itself in various characteristics such as an increased diversity and quantity of beneficial bacteria, improved digestion and absorption of nutrients, reduction of inflammation in the gut, strengthening of the intestinal barrier, and a positive effect on the immune system. Internally, it can also have an influence on the production of short-chain fatty acids, the integrity of the intestinal wall, and the interaction between the gut and other body systems such as the immune system and the central nervous system.

In a preferred embodiment, a recommended daily dose of the composition comprises at least the following ingredients:
- a probiotic component comprising:
   ∘ *Saccharomyces boulardii,*
   ∘ at least three Lactobacillus strains, wherein the Lactobacillus strains are chosen from the group consisting of *Lactobacillus casei, Lactobacillus plantarum, Lactobacillus rhamnosus, Lactobacillus acidophilus,* and *Lactobacillus paracasei,*
- a prebiotic component comprising at least two different sources of soluble fibers, wherein at least one source is of microbial origin,
wherein the composition further comprises hyaluronic acid or a salt thereof.

In one embodiment, a recommended daily dose of the composition comprises the hyaluronic acid or the salt thereof in an amount of at least 1 mg, preferably at least 5 mg, more preferably at least 10 mg, more preferably at least 20 mg.

In one embodiment, a recommended daily dose of the composition comprises the hyaluronic acid or the salt thereof in an amount between 1 and 200 mg, preferably between 5 and 150 mg, preferably between 5 and 150 mg, more preferably between 5 and 100 mg, more preferably between 10 and 50 mg, and even more preferably between 20 and 30 mg.

The hyaluronic acid in the composition can be in the form of hyaluronic acid or a salt thereof. Examples of salts of hyaluronic acid are sodium hyaluronate, potassium hyaluronate, and calcium hyaluronate. In a preferred embodiment of the invention, the composition comprises hyaluronic acid or sodium hyaluronate.

It has been found that when the daily dose of hyaluronic acid is higher than the recommended amount, this can lead to excessive fluid retention, disruption of the gut microbiota, and gastrointestinal discomfort. On the other hand, a dose lower than the recommended amount can result in insufficient hydration of the intestinal wall, a reduced intestinal barrier function, and a decrease in the synergistic effects of the composition. An optimal daily dose of hyaluronic acid ensures the proper hydration and integrity of the intestinal barrier, promotes the balance of the gut microbiota, and maximizes the synergistic benefits of the prebiotic and probiotic components, resulting in improved overall intestinal health and well-being.

In another or further preferred embodiment, a recommended daily dose of the composition comprises at least the following ingredients:
- a probiotic component comprising:
   ∘ *Saccharomyces boulardii,,*
   ∘ at least three Lactobacillus strains, wherein the Lactobacillus strains are chosen from the group consisting of *Lactobacillus casei, Lactobacillus plantarum, Lactobacillus rhamnosus, Lactobacillus acidophilus,* and *Lactobacillus paracasei,* wherein the total amount of *Lactobacillus* in the daily dose is between 10⁸ and 10¹⁰ colony-forming units (CFU),
- a prebiotic component comprising at least two different sources of soluble fibers, wherein at least one source is of microbial origin,
wherein the composition further comprises hyaluronic acid or a salt thereof, wherein a recommended daily dose of the composition comprises the hyaluronic acid or the salt thereof in an amount of at least 5 mg.

The composition comprises, among other things, ingredients that in themselves have already been ascribed a positive role in supporting and improving gut health and gut flora. The composition is optimized such that its components have a synergistic effect and will optimally improve the intestinal health of a subject, preferably a human or an animal. More particularly, it has been found that the composition has an optimal effect on the balance and diversity of the gut microbiota. Moreover, upon daily oral intake, the composition is also found to have a positive effect on intestinal health and inflammation levels. Finally, a test population that took the supplement reported a clear improvement in digestive comfort, immune function, and overall well-being.

Furthermore, it has been found that the composition comprising hyaluronic acid has an optimal effect in the gastrointestinal tract, wherein hyaluronic acid has the ability to absorb water. In the intestine, hyaluronic acid helps to retain water in the extracellular matrix of the intestinal wall, which contributes to the hydration of the tissues. This is important for the health of the intestinal cells, as a well-hydrated environment is essential for their function. Most water absorption in the body takes place in the intestines, after which the water is transported to other parts of the body. A well-hydrated intestinal tissue layer also maintains the integrity of the intestinal barrier, thereby preventing pathogens from entering the body and ensuring that the immune functions of the gut proceed optimally.

In one embodiment, the composition comprises hyaluronic acid or a salt thereof in an amount ranging between 0.5 and 20 wt.%, preferably between 1 and 20 wt.%, more preferably between 1 and 15 wt.%, even more preferably between 1 and 10 wt.%, and even more preferably between 2.5 and 7.5 wt.%.

In one embodiment, a recommended daily dose of the composition comprises the hyaluronic acid or a salt thereof and the probiotic component in a ratio ranging between 15 mg of hyaluronic acid or a salt thereof per 10¹⁰ colony-forming units (CFU) of probiotic component and 30 mg of hyaluronic acid or a salt thereof per 10¹⁰ colony-forming units (CFU) of probiotic component, preferably between 20 mg/10¹⁰ CFU and 30 mg/10¹⁰ CFU, even more preferably between 20 mg/10¹⁰ CFU and 25 mg/10¹⁰ CFU.

It has been found that when the ratio is too high, it can lead to excessive hydration and disruption of the gut microbiota, whereas a ratio that is too low provides insufficient support to the intestinal barrier and reduces the synergistic effects of the composition. By maintaining an optimal ratio, a maximum synergistic effect is achieved, resulting in improved hydration, strengthening of the intestinal barrier, and promotion of a healthy gut microbiota, which leads to an overall improvement in intestinal health and well-being.

In one embodiment, a recommended daily dose of the composition comprises the hyaluronic acid or a salt thereof and the prebiotic component in a weight ratio ranging between 0.1 and 2, preferably between 0.1 and 1.5, preferably between 0.1 and 1, even more preferably between 0.1 and 0.8, and even more preferably between 0.2 and 0.6.

It has been found that when this ratio is too high, it can lead to excessive hydration and disruption of normal intestinal functions, whereas a ratio that is too low provides insufficient support for the prebiotic action, resulting in reduced stimulation of beneficial gut bacteria. By maintaining an optimal ratio, the synergistic effect of hyaluronic acid and prebiotics is maximized, resulting in improved hydration of the intestinal wall, strengthening of the intestinal barrier, and promotion of a healthy and diverse gut microbiota, which leads to an overall improvement of intestinal health and well-being.

In one embodiment, the probiotic component is in a weight ratio to the prebiotic component ranging from 1 to 3. In a further embodiment, the probiotic component is in a weight ratio to the prebiotic component ranging from 1 to 2.5, preferably from 1 to 2, even more preferably from 1.5 to 2.

A balanced ratio between probiotics and prebiotics provides an optimal growth medium for the probiotic bacteria, thereby supporting their survival and growth in the gut. This results in more efficient colonization and better health effects. When this ratio is too high, it can lead to an overabundance of probiotics without a sufficient growth medium, which reduces their growth and survival in the gut.

In one embodiment, the composition comprises a probiotic component comprising Saccharomyces boulardii, and at least three *Lactobacillus* strains, wherein the Lactobacillus strains are chosen from the group consisting of Lactobacillus casei, Lactobacillus plantarum, Lactobacillus rhamnosus, Lactobacillus acidophilus, and Lactobacillus paracasei.

In a preferred embodiment, the probiotic component comprises *Lactobacillus casei, Lactobacillus plantarum, Lactobacillus rhamnosus, Lactobacillus acidophilus,* and *Lactobacillus paracasei* as the *Lactobacillus* strains.

In one embodiment, a recommended daily dose of the composition comprises the *Saccharomyces boulardii* in an amount of at least 5x10⁸ colony-forming units (CFU), preferably at least 10⁹ CFU, more preferably at least 2x10⁹ CFU, more preferably at least 3x10⁹ CFU, more preferably at least 4x10⁹ CFU, more preferably at least 5x10⁹ CFU, even more preferably at least 6x10⁹ CFU, even more preferably at least 7x10⁹ CFU, even more preferably at least 8x10⁹ CFU, even more preferably at least 9x10⁹ CFU, even more preferably at least 10¹⁰ CFU, even more preferably at least 1.1x10¹⁸ CFU, and most preferably at least 1.2x10¹⁰ CFU.

In one embodiment, a recommended daily dose of the composition comprises the *Saccharomyces boulardii* in an amount between 5x10⁸ and 10¹¹ colony-forming units (CFU), more preferably between 10⁹ and 10¹¹ CFU, more preferably between 10⁹ and 5x10¹⁰, preferably between 5x10⁹ and 5x10¹⁰ CFU, more preferably between 6x10⁹ and 5x10¹⁰ CFU, more preferably between 7x10⁹ and 5x10¹⁰ CFU, more preferably between 8x10⁹ and 5x10¹⁰ CFU, more preferably between 9x10⁹ and 5x10¹⁰ CFU, even more preferably between 10¹⁰ and 5x10¹⁰ CFU, even more preferably between 1.1x10¹⁰ and 5x10¹⁸ CFU, and most preferably between 1.2x10¹⁰ and 5x10¹⁸ CFU.

In one embodiment, the composition comprises *Saccharomyces boulardii* in an amount of at least 10⁹ colony-forming units (CFU)/g of composition, preferably at least 5x10⁹ CFU/g, more preferably at least 6x10⁹ CFU/g, more preferably at least 7x10⁹ CFU/g, more preferably at least 8x10⁹ CFU/g, more preferably at least 9x10⁹ CFU/g, more preferably at least 10¹⁰ CFU/g, more preferably at least 2x10¹⁰ CFU/g.

In one embodiment, the composition comprises *Saccharomyces boulardii* in an amount ranging from 10⁹ to 10¹¹ colony-forming units (CFU)/g of composition, preferably between 5x10⁹ and 10¹¹ CFU/g, more preferably between 6x10⁹ and 10¹¹ CFU/g, more preferably between 7x10⁹ and 10¹¹ CFU/g, more preferably between 8x10⁹ and 10¹¹ CFU/g, more preferably between 9x10⁹ and 10¹¹ CFU/g, more preferably between 10¹⁰ and 10¹¹ CFU/g, more preferably between 10¹⁰ and 5x10¹⁰ CFU/g, more preferably between 10¹⁰ and 3x10¹⁰ CFU/g.

In one embodiment, the probiotic component comprises *Saccharomyces boulardii,* in an amount of at least 5x10⁹ colony-forming units (CFU)/g of composition, preferably at least 6x10⁹ CFU/g, more preferably at least 7x10⁹ CFU/g, more preferably at least 8x10⁹ CFU/g, more preferably at least 9x10⁹ CFU/g, more preferably at least 10¹⁰ CFU/g, more preferably at least 2x10¹⁰ CFU/g, more preferably at least 3x10¹⁰ CFU/g.

In one embodiment, the probiotic component comprises *Saccharomyces boulardii,* in an amount ranging from 5x10⁹ to 10¹¹ colony-forming units (CFU)/g of probiotic component, preferably between 6x10⁹ and 10¹¹ CFU/g, more preferably between 7x10⁹ and 10¹¹ CFU/g, more preferably between 8x10⁹ and 10¹¹ CFU/g, more preferably between 9x10⁹ and 10¹¹ CFU/g, more preferably between 10¹⁰ and 10¹¹ CFU/g, more preferably between 1.5x10¹⁰ and 5x10¹⁰ CFU/g, more preferably between 2x10¹⁰ and 5x10¹⁰ CFU/g, more preferably between 3x10¹⁰ and 4x10¹⁰ CFU/g.

In one embodiment, a recommended daily dose of the composition comprises a total amount of *Lactobacillus* strains in an amount ranging from 10⁸ to 10¹⁰ colony-forming units (CFU), preferably between 5x10⁸ and 5x10¹⁰ CFU, preferably between 10⁹ and 10¹⁰, preferably between 2x10⁹ and 9x10⁹ CFU, more preferably between 3x10⁹ and 8x10⁹ CFU, more preferably between 4x10⁹ and 7x10⁹ CFU, more preferably between 4x10⁹ and 6x10⁹ CFU, more preferably between 4.5x10⁹ and 5.5x10⁹ CFU.

In one embodiment, the composition comprises a total amount of *Lactobacillus* strains in an amount ranging from 10⁸ to 5x10¹⁰ colony-forming units (CFU)/g of composition, preferably between 5x10⁸ and 5x10¹⁰ CFU/g of composition, preferably between 10⁹ and 5x10¹⁰ CFU/g, preferably between 2x10⁹ and 4x10¹⁰ CFU/g, more preferably between 3x10⁹ and 3x10¹⁰ CFU/g, more preferably between 4x10⁹ and 2x10¹⁰ CFU/g, more preferably between 5x10⁹ and 1x10¹⁰ CFU/g, more preferably between 6x10⁹ and 1x10¹⁰ CFU/g, more preferably between 7x10⁹ and 1x10¹⁰ CFU/g, more preferably between 8x10⁹ and 9x10⁹ CFU/g, more preferably between 8x10⁹ and 8.5x10⁹ CFU/g.

In one embodiment, the probiotic component comprises a total amount of *Lactobacillus* strains in an amount ranging from 5x10⁸ to 10¹¹ colony-forming units (CFU)/g of probiotic component, preferably between 10⁸ and 10¹¹ CFU/g of probiotic component, preferably between 5x10⁹ and 10¹¹ CFU/g of probiotic component, preferably between 5x10⁹ and 7x10¹⁰ CFU/g, more preferably between 6x10⁹ and 6x10¹⁰ CFU/g, more preferably between 7x10⁹ and 5x10¹⁰ CFU/g, more preferably between 8x10⁹ and 4x10¹⁰ CFU/g, more preferably between 9x10⁹ and 3x10¹⁰ CFU/g, more preferably between 1x10¹⁰ and 2x10¹⁰ CFU/g.

In one embodiment, a recommended daily dose of the composition comprises each *Lactobacillus* strain in an amount ranging from 10⁸ to 10¹⁰ colony-forming units (CFU), preferably between 10⁸ and 5x10⁹, more preferably between 5x10⁸ and 5x10⁹, preferably between 6x10⁸ and 5x10⁹ CFU, more preferably between 7x10⁸ and 4x10⁹ CFU, more preferably between 8x10⁸ and 3x10⁹ CFU, more preferably between 9x10⁸ and 2x10⁹ CFU, more preferably between 9.5x10⁸ and 1.5x10⁹ CFU.

In one embodiment, the composition comprises each *Lactobacillus* strain in an amount ranging from 10⁸ to 10¹⁰ colony-forming units (CFU) per g of composition, preferably between 10⁸ and 5x10⁹, more preferably between 5x10⁸ and 5x10⁹, preferably between 6x10⁸ and 5x10⁹ CFU, more preferably between 7x10⁸ and 4x10⁹ CFU, more preferably between 8x10⁸ and 3x10⁹ CFU/g, more preferably between 9x10⁸ and 2.5x10⁹ CFU/g, more preferably between 1x10⁹ and 2x10⁹ CFU/g.

In one embodiment, the probiotic component comprises each *Lactobacillus* strain in an amount ranging from 10⁸ to 10¹⁰ colony-forming units (CFU) per g of composition, preferably between 10⁸ and 5x10⁹, more preferably between 8x10⁸ and 5x10⁹, preferably between 9x10⁸ and 5x10⁹ CFU, more preferably between 1x10⁹ and 4x10⁹, more preferably between 2x10⁹ and 3.5x10⁹ CFU/g, more preferably between 2.5x10⁹ and 3x10⁹ CFU/g.

In one embodiment, a recommended daily dose of the composition comprises a total amount of *Lactobacillus* and the *Saccharomyces boulardii* in a ratio ranging from 0.5 to 1 (in CFU/CFU), preferably between 0.5 and 1, more preferably between 0.8 and 1, more preferably between 0.85 and 1, more preferably between 0.9 and 1, even more preferably between 0.95 and 1.

It has been found that an optimal ratio is close to 1/1. By maintaining an optimal ratio, the synergistic effect between *Saccharomyces boulardii* and *Lactobacillus* is maximized, which results in an improved balance and diversity of the gut microbiota, increased protection against pathogens, and an overall improvement of intestinal health and well-being.

In one embodiment, the Lactobacillus strains are encapsulated *Lactobacillus* strains. As used herein, "encapsulated *Lactobacillus* strains" and "coated *Lactobacillus* strains" refer to *Lactobacillus* strains that are enveloped in a protective material to ensure or improve their survival and efficacy. An encapsulation can consist of one or more protective layers or coatings.

In one embodiment, the encapsulated *Lactobacillus* are coated in two layers. A double coating provides additional protection to the Lactobacillus strains, thereby further improving their survival and efficacy.

In one embodiment, the composition comprises a prebiotic component that comprises at least two different sources of soluble fibers, and wherein preferably at least one source is of microbial origin. In a preferred embodiment, the composition comprises a prebiotic component that comprises two different sources of soluble fibers, and wherein one source is of microbial origin.

Soluble fibers can be derived from vegetables, fruits, nuts, grains, plants, and microorganisms. Examples of sources of soluble fibers are inulin, pectin, guar gum, psyllium, fructo-oligosaccharides (FOS), galacto-oligosaccharides (GOS), acacia fibers, baobab fibers, beta-glucans, xylo-oligosaccharides (XOS), konjac glucomannan, gum arabic, and resistant starches.

In one embodiment, one of the sources of soluble fibers is beta-glucan. Beta-glucan supports immune function and promotes the growth of beneficial gut bacteria, which contributes to better intestinal health.

In a further embodiment, a recommended daily dose of the composition comprises beta-glucan in an amount ranging from 10 to 100 mg, preferably between 25 and 75 mg, preferably between 40 and 60 mg. This refers to the active beta-glucan.

The beta-glucan can be of microbial origin, such as obtained from yeasts, fungi, or bacteria. Examples are beta-glucan obtained from *Saccharomyces cerevisiae, Aspergillus niger, Ganoderma lucidum* (Reishi), *Lentinula edodes* (Shiitake), and *Agrobacterium;* preferably, the beta-glucan is obtained from *Saccharomyces cerevisiae.*

In a preferred embodiment, the beta-glucan is 1,3/1,6 beta-glucan obtained from *Saccharomyces cerevisiae.* In the context of the composition, which also includes hyaluronic acid, 1,3/1,6 beta-glucan proves to be particularly useful. Hyaluronic acid improves the hydration of the intestinal wall and strengthens the integrity of the gut barrier, which is essential for preventing pathogenic invasion and promoting the absorption of nutrients. Together, beta-glucan and hyaluronic acid provide a synergistic effect: while beta-glucan strengthens immune function and promotes a healthy gut flora, hyaluronic acid ensures a well-hydrated and strong gut barrier. This combined effect results in an overall improvement of intestinal health, a strengthened immune response, and an increased well-being of the individual.

In one embodiment, one of the sources of soluble fibers is baobab fiber and/or acacia fiber, preferably baobab fiber and acacia fiber. Baobab fibers and acacia fibers are soluble fibers that promote the growth of beneficial gut bacteria and contribute to better digestion and intestinal health.

In a further embodiment, a recommended daily dose of the composition comprises a total amount of baobab fiber and acacia fiber in an amount ranging from 1 to 50 mg, preferably between 1 and 25 mg, preferably between 5 and 15 mg.

In a further embodiment, a recommended daily dose of the composition comprises a total amount of soluble fibers in an amount ranging from 10 to 100 mg, preferably between 25 and 100 mg, preferably between 50 and 70 mg.

In one embodiment, the composition further comprises artichoke extract. Promotes digestion and helps to eliminate toxins. Moreover, the artichoke extract also ensures that the composition has a cholesterol-controlling or cholesterol-lowering effect.

In a further embodiment, a recommended daily dose of the composition comprises artichoke extract in an amount ranging from 10 to 100 mg, preferably between 25 and 75 mg, preferably between 40 and 75 mg. In a further or other embodiment, a recommended daily dose of the composition comprises artichoke extract in an amount of at least 50 mg.

In one embodiment, the components of the composition are in a single dosage form. In another embodiment, the components of the composition are in separate dosage forms. In the latter case, one or more components can be in separate dosage forms, as for example when the probiotic component and the prebiotic component are in separate dosage forms, wherein the other components, such as the hyaluronic acid, are then located in one of these two dosage forms.

In a preferred embodiment, the components of the composition are in a single dosage form, wherein the dosage form is preferably an oral dosage form.

Examples of an oral dosage forms include, but are not limited to, a tablet, capsule, powder that can be dispersed in a beverage, a liquid such as a solution, suspension or emulsion, a soft gel/chewable capsule, a lozenge, a paste, elixir, syrup, drops, bar, beverage, chewable tablet, a chewable stick, or another suitable dosage form known in **the art.** Preferably, the composition is formulated in powder form, as a tablet, a capsule, or a granule. Furthermore, the oral dosage forms can be formulated for immediate release, extended release, or delayed release.

In a preferred embodiment, the composition is a capsule and a daily dose is preferably ranging from 100 to 1000 mg, preferably between 250 and 750 mg, more preferably between 500 and 700 mg, even more preferably between 550 and 650 mg. The daily dose may be in one or more capsules.

In another preferred embodiment, the composition is a powder and a daily dose is preferably ranging from 100 to 1000 mg, preferably between 250 and 750 mg, more preferably between 400 and 600 mg, even more preferably between 450 and 550 mg.

In particular, a recommended daily dose of the composition comprises at least the following ingredients:
- a probiotic component comprising:
   o *Saccharomyces boulardii,* in an amount of at least 10⁹ colony-forming units (CFU),
   ∘ *Lactobacillus* strains, wherein the *Lactobacillus* strains are *Lactobacillus casei, Lactobacillus plantarum, Lactobacillus rhamnosus, Lactobacillus acidophilus,* and *Lactobacillus paracasei,* wherein the total amount of *Lactobacillus* in the daily dose is between 10⁸ and 10¹⁰ colony-forming units (CFU),
- a prebiotic component comprising at least two different sources of soluble fibers, wherein at least one source is of microbial origin,
wherein the composition further comprises hyaluronic acid and/or a salt thereof, wherein a recommended daily dose of the composition comprises the hyaluronic acid and/or the salt thereof in an amount of at least 5 mg.

In another or further preferred embodiment, a recommended daily dose of the composition comprises at least the following ingredients:
- a probiotic component comprising:
   *o Saccharomyces boulardii,* in an amount of at least 10⁹ colony-forming units (CFU),
   ∘ at least three Lactobacillus strains, wherein the Lactobacillus strains are chosen from the group consisting of *Lactobacillus casei, Lactobacillus plantarum, Lactobacillus rhamnosus, Lactobacillus acidophilus,* and *Lactobacillus paracasei,* wherein the total amount of *Lactobacillus* in the daily dose is between 10⁸ and 10¹⁰ colony-forming units (CFU),
- a prebiotic component comprising beta-glucan, baobab fiber, and acacia fiber as a source of soluble fibers, wherein the beta-glucan is of microbial origin,
wherein the composition further comprises hyaluronic acid and/or a salt thereof, wherein a recommended daily dose of the composition comprises the hyaluronic acid and/or the salt thereof in an amount of at least 5 mg.

In particular, a recommended daily dose of the composition comprises at least the following ingredients:
- a probiotic component comprising:
   o *Saccharomyces boulardii,* in an amount of at least 10⁹ colony-forming units (CFU),
   ∘ at least three Lactobacillus strains, wherein the Lactobacillus strains are chosen from the group consisting of Lactobacillus casei, Lactobacillus plantarum, Lactobacillus rhamnosus, Lactobacillus acidophilus, and Lactobacillus paracasei, wherein the total amount of *Lactobacillus* in the daily dose is between 10⁸ and 10¹⁰ colony-forming units (CFU),
- a prebiotic component comprising at least two different sources of soluble fibers, wherein at least one source is of microbial origin, wherein the total amount of soluble fibers in the daily dose ranges from 25 to 100 mg,
wherein the composition further comprises hyaluronic acid and/or a salt thereof, wherein a recommended daily dose of the composition comprises the hyaluronic acid and/or the salt thereof in an amount of at least 5 mg.

In particular, a recommended daily dose of the composition comprises at least the following ingredients:
- a probiotic component comprising:
   ∘ *Saccharomyces boulardii,*
   ∘ *Lactobacillus* strains, wherein the Lactobacillus strains are *Lactobacillus casei, Lactobacillus plantarum, Lactobacillus rhamnosus, Lactobacillus acidophilus* and *Lactobacillus paracasei,*
- a prebiotic component comprising at least two different sources of soluble fibers, wherein at least one source is of microbial origin,
wherein the composition further comprises hyaluronic acid and/or a salt thereof, wherein a recommended daily dose of the composition comprises the hyaluronic acid and/or a salt thereof and the probiotic component in a ratio ranging between 15 mg of hyaluronic acid or a salt thereof per 10¹⁰ colony-forming units (CFU) of the probiotic component and 30 mg of hyaluronic acid or a salt thereof per 10¹⁰ colony-forming units (CFU) of the probiotic component, and wherein optionally a recommended daily dose of the composition comprises the hyaluronic acid and/or a salt thereof and the prebiotic component in a weight ratio ranging between 0.1 and 1.

Compositions according to the present disclosure may additionally and/or optionally further comprise one or more excipients. These excipients are selected from the following group: microcrystalline cellulose, crystalline cellulose, lactose, corn starch, sucrose, glucose; binders such as tragacanth, gum arabic, corn starch, gelatin, polyvinyl alcohol, polyvinyl ether, ethylcellulose, methylcellulose, shellac, hydroxypropyl cellulose, hydroxypropyl starch, polyvinylpyrrolidone; swelling agents such as corn starch, pregelatinized starch, alginic acid, dextrin; lubricants such as magnesium stearate; flow-enhancing agents such as fine silicon dioxide; lubricants such as glyceryl fatty acid ester, magnesium stearate, talc, polyethylene glycol, silica, hydrogenated vegetable oil; sweeteners such as sucrose, lactose, aspartame, acesulfame-K, sucralose, monatin, stevia, saccharin and the like; flavors to be used for various food products such as peppermint, vanilla flavor, cherry, raspberry ketone and the like; preservatives such as para-hydroxybenzoates (parabens), chlorobutanol, benzyl alcohol, sorbic acid; an antioxidant, such as sulfite, ascorbic acid, vitamin E, butylhydroxytoluene, sodium sulfite; and/or coating agents such as shellac, sucrose, gelatin and hydroxypropyl cellulose.

Compositions according to the present disclosure may additionally and/or optionally further comprise phytochemical molecules derived from at least one of the following sources: blueberry, cranberry, raspberry, cherry, mulberry, maqui berry, purple corn, strawberry, grapes, gooseberries, black currants, grape must, cocoa beans, coffee beans, pine bark, cardamom, cinnamon bark, ginseng, astragalus, rhodiola, garcinia, ginger, ginkgo, citrus fruits, grape skins, grape seeds, hawthorn, apple, olive, orange, lemon, pepper, soy, mango, tea leaves, tomato, cabbage, black rice, bitter lemon, stevia, lo han, goji (wolfberry), sea buckthorn, kudzu, clove, hemp, cassia, magnolia, nutmeg, jujube, honeysuckle, poria, bellflower, lotus, basil, sesame, angelica, cimicifuga, epimedium, schisandra, salvia, licorice, privet, ophiopogonis, aloe, dodder, fenugreek, gotu kola, guarana, purslane and tribulus.

Compositions according to the present description may additionally and/or optionally further comprise one or more extra natural extracts, selected from one or more plants of the following genera: Origanum Thymus, Lavandula, Salvia, Melissa, Cuminum, Petroselinum, Calendula, Tagetes, Boswellia, Sambucus, Copaifera, Allium, Symphytum, Punica, Euterpe, Sophora, Rheum, Fagopyrum, Camellia, Coptis, Hydrastis, Mahonia, Phellodendron, Berberis, Xanthorhiza, Lonicera, Vaccinium, Cinnamomum, Vitis, Terminalia, Pinus, Albizia, Melia, Salvadora, Paullinia, Commiphora, Juglans, Scutellaria, and Magnolia.

More specifically, the additional natural extract used in the compositions described herein can be extracted from plants of the following species: Origanum vulgare, Origanum onites, Origanum majorana, Origanum heracleoticum, Thymus vulgaris L, Thymus citriodorus, Thymus pulegioides, Thymus x herba-barona , Thymus serpyllum, Lavandula angustifolia / officinalis, Lavandula stoechas, Lavandula dentata, Lavandula x intermedia, Lavandula multifida, Salvia officinalis, Salvia divinorum, Salvia apiana, Melissa officinalis, Cuminum cyminum, Petroselinum crispum, Calendula arvensis, Calendula maderensis, Calendula officinalis, Tagetes erecta, Tagetes minuta, Tagetes patula, Boswellia sacra, Boswellia frereana, Boswellia serrata, Boswellia papyrifera, Sambucus nigra, Sambucus melanocarpa, Sambucus racemosa, Copaifera langsdorfii, Curcuma longa, Allium sativum, Symphytum officinale, Punica granatum, Euterpe oleracea, Sophora flavescens, Rheum rhabarbarum, Rheum rhaponticum, Fagopyrum esculentum, Camellia sinensis, Coptis teeta, Hydrastis canadensis, Mahonia aquifolium, Phellodendron amurense, Berberis vulgaris, Xanthorhiza simplicissima, Lonicera caprifolium, Vaccinium macrocarpon, Cinnamomum zeylanicum Nees, Cinnamomum verum, Vitis Vinifera, Terminalia Bellerica, Pinus Pinaster, Albizia Lebbek, Melia Azadirachta, Salvadora persica, Paullinia cupana, Piper betle, Commiphora myrrha, Juglans regia, Scutellaria baicalensis and Lombardia officinalis.

Additional natural extracts that may be useful together with previously described active ingredients may also be selected from one or more of the following natural extracts (common name included first, not italicized, followed by formal italicized name(s)): achyranthes, Achyranthes aspera, aloe, Aloe spp., including A. barbadensis, A. ferox and A. vera, anise, Pimpinella anisum, aristolochia, Aristolochia bracteolata, arnica, Arnica spp., including A. fulgens, banyan, Ficus bengalensis, bakula, Mimusops elengi, basil, Ocimum basilicum and O. minimum, betel, Piper betle, camphor, Cinnamomum camphora, catechu, Acacia catechu, greater celandine, Chelidonium spp., chamomile, Matricaria chamomilla, chebula, Terminalia chebula, Chinese skullcap, Scutellaria baicalensis, cinnamon, Cinnamomum lourerii and C. zeylandicum, citrus, Citrus spp., including C. aurantifolia, C. aurantium, C. limonum and C. sinensis, dill, Anethum spp., including A. graveolens and A. sowa, echinacea (coneflower), Echinacea pallida, eucalyptus, Eucalyptus globulus, fennel, Foeniculum vulgare, gardenia, Gardenia jasminoides, grape, Vitis vinifera, hop, Humulus lupulus, houttuynia, Houttuynia cordata, Indian mulberry, Morinda citrifolia, juniper, Juniperus communis, lemongrass, Cymbopogon spp., including C. citratus and C. flexuosus, licorice, Glycyrrhiza spp., including G. glabra and G. uralensis, long pepper (pipli), Piper longum, madhuca, Madhuca longifolia, magnolia, Magnolia officinalis, marigold, Calendula officinalis, mastic, Pistacia lentiscus, sweet clover, Melilotus officinalis, yarrow, Achillea millefolium, myrrh, Commiphora spp., neem (margosa), Azadirachta indica, neroli (bitter orange blossom), Citrus aurantium, nutmeg, Myristica fragrans, oak gall, Quercus infectoria, parsley, Petroselinum sativum, peelu, Salvadora persica, peppermint, Mentha piperita, pine, Pinus spp., including P. palustris and P. sylvestris, pomegranate, Punica granatum, prickly acacia (babul), Acacia nilotica, rhatany, Krameria spp., including K. argentea and K. triandra, rosemary, Rosmarinus officinalis, saffron, Crocus sativus, sage, Salvia spp., including S. lavendulaefolia, S. officinalis and S. triloba, sandalwood, Santalum spp., including S. album and S. spicatum, spearmint, Mentha spicata, spilanthes (akarkara), Spilanthes calva, star anise, Illicium verum, tea (including green tea and oolong tea), Camellia sinensis, thyme, Thymus spp., including T. serpyllum and T. vulgaris, tomar (prickly ash), Zanthoxylum armatum, tulsi (holy basil), Ocimum sanctum, usnea, Usnea barbata, vajradanti, Potentilla fulgens, walnut, Juglans regia, wintergreen, Gaultheria procumbens, and mixtures thereof.

As discussed herein, the additional natural extracts can be derived from or be based on compounds or extracts isolated from plants.

Compositions and formulations according to the present disclosure may additionally and/or optionally further comprise one or more vitamins selected from the group consisting of: vitamin A, vitamin C, vitamin B1, vitamin B2, vitamin B5, vitamin B6, vitamin B12, vitamin D, vitamin E, and/or derivatives thereof, folic acid, biotin.

Compositions and formulations according to the present invention may additionally and/or optionally further comprise one or more carrier materials, such as corn starch, acacia, gelatin, malt, tragacanth, microcrystalline cellulose, kaolin, dicalcium phosphate, calcium carbonate, sodium chloride or alginic acid; disintegrants including microcrystalline cellulose or alginic acid; binders including acacia, methylcellulose, ethylcellulose, sodium carboxymethylcellulose, polyvinylpyrrolidone or hydroxypropyl methylcellulose; and lubricants such as magnesium stearate, stearic acid, silicone fluid, talc, oils, waxes or colloidal silica.

Compositions and formulations according to the present invention may additionally and/or optionally further comprise one or more mixtures of acids (such as citric acid, tartaric acid, malic acid and fumaric acid or a combination thereof) and carbonates such as sodium, potassium bicarbonate or carbonate; water-soluble binders (starches, natural gums, cellulose gum, microcrystalline cellulose, methylcellulose, cellulose ethers, ethylcellulose, sodium carboxymethylcellulose, gelatin, dextrose, lactose, sucrose, sorbitol, mannitol, polyethylene glycol, polyvinylpyrrolidone, pectins, alginates, polyacrylamides, polyvinyloxazolidone, polyvinyl alcohols and mixtures thereof) and/or lubricants (sodium benzoate, polyethylene glycol, L-leucine, adipic acid and combinations thereof).

Compositions and formulations according to the present invention may additionally and/or optionally further comprise one or more other ingredients, including, for example, flavoring agents, fillers, surfactants, coloring agents and sweeteners.

Compositions and formulations according to the present invention may additionally and/or optionally further comprise one or more aqueous or non-aqueous carriers, diluents, fillers, binders, wetting agents, disintegrants, dissolution retardants, absorption accelerators, humectants, absorbents or lubricants, solvents or carriers, including water-ethanol, polyols (propylene glycol, polyethylene glycol or glycerol), suitable mixtures thereof, vegetable oils (such as olive oil) and organic esters such as ethyl oleate.

Furthermore, the compositions according to the present disclosure may comprise stabilizers, solubilizing agents, suspending agents, emulsifiers, soothing agents, buffers, preservatives, isotonic agents or antibacterial and antifungal agents.

The composition is particularly advantageous for use in humans, but can also be used in animals, and especially mammals.

In a second and further aspect, the present invention relates to, on the one hand, a method for improving intestinal health and, on the other hand, a method for promoting a healthy gut flora, wherein in each case a composition according to one of the embodiments as described above is taken orally. In a preferred embodiment, the composition is taken once or twice daily with a liquid, more preferably the composition is dissolved or dispersed in a liquid for ingestion. In a further preferred embodiment, the composition is administered in capsule or tablet form and taken with a liquid.

The composition has a positive influence on the intestinal barrier and the microbial balance in the intestines, which contributes to an overall improvement of the intestinal health and the immune system of the subject who takes the composition long-term (at least three months). Preferably, the composition is taken with a liquid, more preferably dissolved in a liquid for ingestion. In particular, the subject will be a human or a mammal.

In one embodiment, the daily dose or 1 serving of the composition comprises:
- a probiotic component comprising:
   ∘ *Saccharomyces boulardii,* in an amount between 10⁹ and 10¹⁰ colony-forming units (CFU),
   ∘ at least three Lactobacillus strains, wherein the Lactobacillus strains are chosen from the group consisting of Lactobacillus casei, Lactobacillus plantarum, Lactobacillus rhamnosus, Lactobacillus acidophilus, and Lactobacillus paracasei, wherein the total amount of Lactobacillus in the daily dose is between 10⁸ and 10¹⁰ colony-forming units (CFU),
- a prebiotic component comprising at least two different sources of soluble fibers, wherein at least one source is of microbial origin,
- hyaluronic acid and/or a salt thereof, wherein a recommended daily dose of the composition comprises the hyaluronic acid and/or the salt thereof in an amount of at least 5 mg.

In one embodiment, the daily dose or 1 serving of the composition comprises:
- a probiotic component comprising:
   ∘ *Saccharomyces boulardii,* in an amount of at least 10⁹ colony-forming units (CFU),
   ∘ at least three Lactobacillus strains, wherein the Lactobacillus strains are chosen from the group consisting of Lactobacillus casei, Lactobacillus plantarum, Lactobacillus rhamnosus, Lactobacillus acidophilus, and Lactobacillus paracasei, wherein the total amount of Lactobacillus in the daily dose is between 10⁸ and 10¹⁰ colony-forming units (CFU),
- a prebiotic component comprising beta-glucan, baobab fiber, and acacia fiber as a source of soluble fibers, wherein the beta-glucan is of microbial origin, and wherein the total amount of soluble fibers in the daily dose is between 25 and 100 mg,
- hyaluronic acid and/or a salt thereof, wherein a recommended daily dose of the composition comprises the hyaluronic acid and/or the salt thereof in an amount of at least 5 mg.

In what follows, the invention will be described by way of non-limiting examples illustrating the invention, and which are not intended to and should not be interpreted as limiting the scope of the invention.

### EXAMPLES

### Example 1

Table 1 shows compositions according to an embodiment of the present invention:

| **Ingredient** | **Unit** | **Per g of composition** | **Per daily dose** |
|---|---|---|---|
| Saccharomyces boulardii | CFU | 10¹⁰ | 6*10⁹ |
| L. acidophilus | CFU | 1,67*10⁹ | 1*10⁹ |
| L. casei | CFU | 1,67*10⁹ | 1*10⁹ |
| L. plantarum | CFU | 1,67*10⁹ | 1*10⁹ |
| L. paracasei | CFU | 1,67*10⁹ | 1*10⁹ |
| L. rhamnosus | CFU | 1,67*10⁹ | 1*10⁹ |
| Baobab & Acacia fiber | mg | 1.67 | 10 |
| Beta-glucan (active) | mg | 83.33 | 50 |
| Hyaluronic acid | mg | 41.67 | 25 |
| Artichoke extract 15:1 | mg | 83.33 | 50 |
| HPMC | mg | 157.80 | 95 |

### Example 2

Ten human volunteers ingest a composition as shown in Example 1 for 6 months. In addition, a placebo group, also consisting of 10 volunteers, ingests a placebo. The placebo has a similar consistency and taste to the effective composition but contains no active ingredients. Each group consists of 5 women and 5 men, over the age of 40. Each volunteer ingests the composition or the placebo in the morning with water. On day 0, the participants are subjected to an analysis to determine intestinal health and the composition of the gut flora. This analysis includes, inter alia, a blood, urine, and fecal analysis, wherein biomarkers linked to intestinal health are examined. A DNA analysis of the gut flora examines both the presence of different bacterial species and the relative abundance of beneficial and harmful bacteria. During the test, participants are subjected to the same tests bimonthly. After six months, the group that ingested the composition according to Example 1 shows a significant measured improvement in intestinal health, as well as an improvement in the diversity and balance of the gut flora.

### Example 3

The setup from Example 2 is repeated, but now the placebo group ingests the composition from Example 1 without hyaluronic acid, in amounts equal to those from the composition of Example 1. The group ingesting the complete composition shows a significant improvement in intestinal health and diversity of the gut flora compared to the other group.

### Example 4

Hyaluronic acid plays a mediating role in adhesion to cells, and soluble fibers support the growth and activity of beneficial gut microbiota.

In this experiment, the effect of hyaluronic acid in combination with beta-glucans, Inavea Baobab, acacia, and artichoke, present in the composition according to an embodiment of the invention, was investigated. The objective was to evaluate whether the addition of these substances improves the colonization potential of the bacterial strains and yeast strains in vitro.

Two conditions were tested: (1) Lactobacillus and Saccharomyces alone and (2) a composition according to an embodiment of the invention (Lactobacillus and Saccharomyces with hyaluronic acid and soluble fibers). Each formulation was plated on a suitable growth medium and incubated for 1 week at 37 °C. After incubation, the colony-forming units (CFUs) were counted to assess microbial growth.

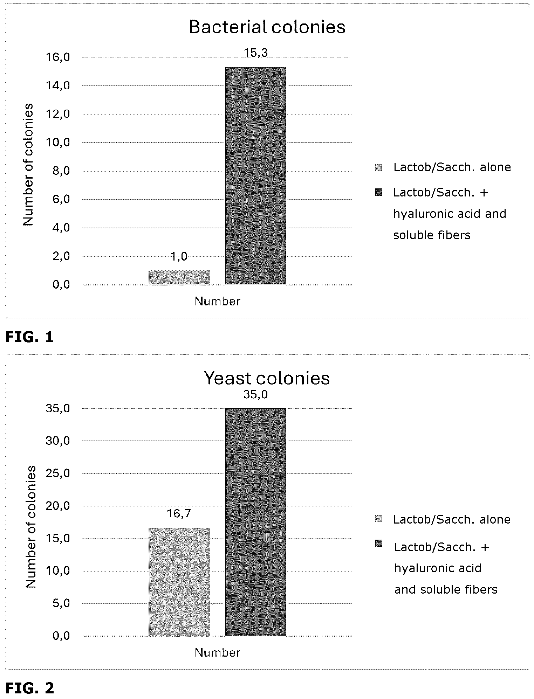

**Figure 1** shows a comparison of the average number of Lactobacillus colony-forming units (CFUs) between the composition according to an embodiment of the invention and the condition without hyaluronic acid and soluble fibers. The composition according to an embodiment of the invention showed a significantly higher average count (15.3 CFUs) compared to the fiber- and hyaluronic acid-free condition (1.0 CFUs), which indicates improved bacterial growth in the presence of hyaluronic acid with soluble fibers.

**Figure 2** shows a comparison of the average number of Saccharomyces colony-forming units (CFUs) between the composition according to an embodiment of the invention and the situation without hyaluronic acid and fibers. The composition according to an embodiment of the invention resulted in a higher average number of yeast colonies (35.0 CFUs) compared to 16.7 CFUs without hyaluronic acid and soluble fibers, indicating improved yeast growth thanks to the added hyaluronic acid and prebiotic fibers.

The composition according to an embodiment of the invention showed improved microbial growth compared to the hyaluronic acid- and fiber-free condition. The addition of hyaluronic acid with soluble fibers such as beta-glucans, Inavea Baobab, acacia, and artichoke in the composition according to an embodiment of the invention improved the in vitro growth of Lactobacillus and Saccharomyces. These findings support the role of hyaluronic acid and soluble fibers in promoting the colonization and potential efficacy of the composition according to an embodiment of the invention.

The present invention should not be construed as being limited to the embodiments described above and certain modifications or changes may be added to the examples described without having to re-evaluate the appended claims.

## Claims

1. A composition for use in supporting and/or improving gut health and gut flora, wherein a recommended daily dose of the composition comprises at least the following ingredients:
- a probiotic component comprising:
∘ *Saccharomyces boulardii,* in an amount of at least 10⁹ colony-forming units (CFU),
∘ at least three Lactobacillus strains, wherein the *Lactobacillus* strains are chosen from the group consisting of *Lactobacillus casei, Lactobacillus plantarum, Lactobacillus rhamnosus, Lactobacillus acidophilus,* and *Lactobacillus paracasei,* wherein the total amount of Lactobacillus in the daily dose is between 10⁸ and 10¹⁰ colony-forming units (CFU),
- a prebiotic component comprising at least two different sources of soluble fibers, wherein at least one source is of microbial origin,
**characterized in that** the composition further comprises hyaluronic acid and/or a salt thereof, wherein a recommended daily dose of the composition comprises the hyaluronic acid and/or the salt thereof in an amount of at least 5 mg.

2. The composition for use according to claim 1, wherein the composition comprises hyaluronic acid and/or a salt thereof, wherein a recommended daily dose of the composition comprises the hyaluronic acid and/or the salt thereof in an amount between 5 and 150 mg.

3. The composition for use according to claim 1 or 2, wherein the weight ratio of the probiotic component to the prebiotic component is between 1 and 3.

4. The composition for use according to any one of the preceding claims, wherein a recommended daily dose of the composition comprises the hyaluronic acid or a salt thereof and the probiotic component in a ratio ranging from 15 mg of hyaluronic acid or a salt thereof per 10¹⁰ colony-forming units (CFU) of the probiotic component to 30 mg of hyaluronic acid or a salt thereof per 10¹⁰ colony-forming units (CFU) of the probiotic component.

5. The composition for use according to any one of the preceding claims, wherein the total daily dose (CFU) *Lactobacillus* in the composition is related to the total daily dose (CFU) *Saccharomyces boulardii* by a ratio between 0.8 and 1.

6. The composition for use according to any one of the preceding claims, wherein the composition comprises *Lactobacillus casei, Lactobacillus plantarum, Lactobacillus rhamnosus, Lactobacillus acidophilus* and *Lactobacillus paracasei.*

7. The composition for use according to any one of the preceding claims, wherein the composition comprises *Saccharomyces boulardii* in an amount between 10⁹ and 10¹¹ colony-forming units (CFU)/g of the composition.

8. The composition for use according to any one of the preceding claims, wherein the composition comprises each *Lactobacillus* in an amount between 10⁸ and 10¹⁰ CFU/g composition.

9. The composition for use according to any one of the preceding claims, wherein the composition comprises a total amount of *Lactobacillus* in an amount between 10⁹ and 10¹¹ CFU/g composition.

10. The composition for use according to any one of the preceding claims, wherein the composition further comprises artichoke extract, wherein a recommended daily dose of the composition comprises artichoke extract in an amount of at least 50 mg.

11. The composition for use according to any one of the preceding claims, wherein a source of soluble fibers is beta-glucan, preferably 1,3/1,6 beta-glucan obtained from *Saccharomyces cerevisiae.*

12. The composition for use according to claim 11, wherein a recommended daily dose of the composition comprises beta-glucan in an amount between 10 and 100 mg.

13. The composition for use according to any one of the preceding claims, wherein a source of soluble fibers is baobab fiber and/or acacia fiber.

14. The composition for use according to any one of the preceding claims, wherein the Lactobacillus strains are encapsulated *Lactobacillus* strains.

15. The composition for use according to any one of the preceding claims, wherein the composition is an oral dosage form, preferably by means of one or more capsules.
